(19) **Europäisches Patentamt / European Patent Office / Office européen des brevets**

(11) **EP 2 890 294 B1**

(12) **FASCICULE DE BREVET EUROPEEN**

(45) Date de publication et mention de la délivrance du brevet:
**20.09.2017 Bulletin 2017/38**

(51) Int Cl.:
***A61B 5/053*** (2006.01)  ***A61B 5/00*** (2006.01)
***A61N 1/20*** (2006.01)

(21) Numéro de dépôt: **13756400.1**

(22) Date de dépôt: **27.08.2013**

(86) Numéro de dépôt international:
**PCT/EP2013/067678**

(87) Numéro de publication internationale:
**WO 2014/033105 (06.03.2014 Gazette 2014/10)**

(54) **SYSTEME D'ANALYSE ELECTROPHYSIOLOGIQUE AMELIORE**

VERBESSERTES ELEKTROPHYSIOLOGISCHES ANALYSESYSTEM

IMPROVED ELECTROPHYSIOLOGICAL ANALYSIS SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.08.2012 FR 1258037**

(43) Date de publication de la demande:
**08.07.2015 Bulletin 2015/28**

(73) Titulaire: **Impeto Medical**
**75014 Paris (FR)**

(72) Inventeurs:
• **KHALFALLAH, Kamel**
**F-75013 Paris (FR)**
• **BRUNSWICK, Philippe**
**F-75014 Paris (FR)**

(74) Mandataire: **Regimbeau**
**20, rue de Chazelles**
**75847 Paris Cedex 17 (FR)**

(56) Documents cités:
**FR-A1- 2 912 893      US-A1- 2013 053 673**

• **DOMINIQUE HUBERT ET AL: "Abnormal electrochemical skin conductance in cystic fibrosis", JOURNAL OF CYSTIC FIBROSIS, vol. 10, no. 1, 1 janvier 2011 (2011-01-01), pages 15-20, XP055063921, ISSN: 1569-1993, DOI: 10.1016/j.jcf.2010.09.002**
• **GIN H ET AL: "Non-invasive and quantitative assessment of sudomotor function for peripheral diabetic neuropathy evaluation", DIABETES & METABOLISM, PARIS, AMSTERDAM, NL, vol. 37, no. 6, 4 mai 2011 (2011-05-04), pages 527-532, XP028338382, ISSN: 1262-3636, DOI: 10.1016/J.DIABET.2011.05.003 [extrait le 2011-06-02] cité dans la demande**

**Description**

DOMAINE DE L'INVENTION

L'invention concerne de manière générale le domaine de l'analyse électrophysiologique du corps humain, en vue par exemple de détecter des pathologies.
L'invention est notamment applicable à l'évaluation de la fonction sudorale du corps humain.

ART ANTERIEUR

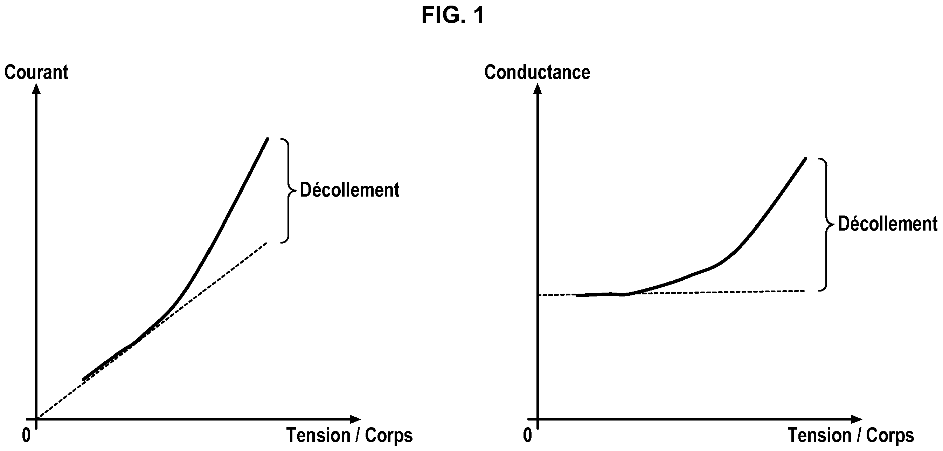

La Demanderesse a déjà proposé dans le brevet FR 2 912 893 un système d'analyse électrophysiologique comprenant une série d'électrodes destinées à être positionnées en différentes régions du corps d'un sujet, une source de tension continue, adaptée pour générer des créneaux de tension continue ajustable, et un circuit de commutation, agencé pour sélectivement relier une paire d'électrodes dites actives à la source de tension, lesdites électrodes actives constituant une anode et une cathode, et pour connecter au moins une autre électrode en haute impédance.
La tension appliquée par la source de tension sur les électrodes permet de générer dans la couche externe de la peau un courant électrophysiologique dont l'étude de certaines caractéristiques peut indiquer l'existence de pathologies ou de prédispositions pathologiques.
Notamment, et en référence à la figure 1, le courant généré dans la peau par l'application d'une tension permet d'étudier le comportement électrique des glandes sudorales du sujet, par la mesure de la conductance de ces glandes. Cette conductance, qui correspond à la pente de la courbe tension-courant, pour les plus faibles tensions (voir demande de brevet FR1160601), varie en fonction de l'état de santé du sujet.
Ainsi par exemple, une faible pente de la courbe tension-courant peut être un indice, chez un sujet diabétique, d'une neuropathie diabétique, comme décrit dans le document de Gin H, et al. « Non-invasive and quantitative assessment of sudomotor function for peripheral diabetic neuropathy evaluation. Diabetes Metab (2011), doi:10.1016/j.diabet.2011.05.003 ».
On a constaté également que la courbe tension-courant présente, pour les niveaux de tension les plus faibles, une portion linéaire, puis un décollement non-linéaire à des niveaux plus importants, voir figure 1. Un décollement correspondant se produit pour la conductance de la peau en fonction du niveau de tension imposé au corps.
Ce décollement ne se produit pas de la même manière pour différents types de sujets, en fonction d'une éventuelle maladie dont ils sont atteints, de sorte que l'étude de ce décollement chez un sujet pourrait permettre d'en déduire des informations sur son état de santé.
Cependant, ce décollement ne survient pas, d'une personne à l'autre, à l'application d'une même tension ou pour un même courant ; au contraire, les inventeurs ont observé que son apparition dépendait du potentiel atteint par le corps et plus précisément de la différence de potentiel de part et d'autre de la glande, c'est-à-dire entre l'électrode et le corps.
Il convient donc de déterminer ce décollement, pour tous les sujets, à la même différence de potentiel entre l'électrode et le corps.
Or les systèmes actuels permettent seulement d'avoir accès à la différence de potentiel entre l'électrode et le potentiel atteint par le corps et ne peuvent pas imposer à priori cette différence de potentiel, de sorte que les mesures ne sont pas effectuées pour une différence de potentiel constante. Ces systèmes ne permettent donc pas d'exploiter pleinement la courbe tension-courant au niveau de son décollement pour obtenir des informations sur l'état de santé du sujet.

RESUME DE L'INVENTION

Le but de la présente invention est de pallier les insuffisances décrites ci-avant, en proposant un système permettant d'étudier le décollement de la courbe des glandes sudorales de différents sujets pour des différences de potentiel identiques.
A cet égard, l'invention a pour objet un système d'analyse électrophysiologique comprenant :

une série d'électrodes destinées à être placées en différentes régions du corps humain,
une source de tension continue,
un dispositif de commande, adapté pour sélectivement appliquer à une paire d'électrodes dites actives des créneaux de tension continue générés par la source de tension, lesdites électrodes actives constituant une anode et une cathode, et pour connecter au moins une autre électrode, dite passive, en haute impédance, ladite électrode servant à mesurer le potentiel atteint par le corps, et
un dispositif de mesure agencé pour relever des données représentatives du courant à la cathode et des potentiels sur au moins certaines électrodes connectées en haute impédance en réponse à l'application des créneaux, lesdites données permettant de déterminer une valeur de la conductance électrochimique de la peau,
le système étant caractérisé en ce qu'il comprend en outre un dispositif de pilotage de la différence entre le potentiel de l'anode et le potentiel atteint par le corps, en fonction de la tension délivrée par la source de tension continue, et en ce que le dispositif de pilotage comprend un circuit amplificateur entre la source de tension continue et l'une des électrodes actives, avec une boucle de rétroaction contrôlant son gain.

Avantageusement, mais facultativement, le système selon l'invention peut en outre comprendre au moins l'une des caractéristiques suivantes :

- l'anode est connectée à la masse, et le circuit amplificateur comprend un amplificateur opérationnel dont :

    ◦ l'entrée inverseuse reçoit le potentiel atteint par le corps,
    ◦ l'entrée non-inverseuse est connectée à une source de tension continue négative, et
    ◦ la sortie est connectée à la cathode.

- la boucle de rétroaction du circuit amplificateur comprend un soustracteur recevant sur ses entrées le potentiel de l'anode et le potentiel atteint par le corps, et le circuit amplificateur à boucle de rétroaction comprend un amplificateur opérationnel dont :

    ◦ l'entrée inverseuse est connectée à la sortie du soustracteur,
    ◦ l'entrée non-inverseuse est connectée à une source de tension continue positive, et
    ◦ la sortie est connectée à l'anode.

- Le système comprend en outre un dispositif pour déterminer le potentiel atteint par le corps en fonction des potentiels des électrodes connectées en haute impédance.
- le soustracteur présente une impédance d'entrée suffisamment forte, pour que les courants d'entrée dans ledit soustracteur ne perturbent pas les mesures.
- Le système comprend une première résistance de mesure branchée en série entre la source de tension et l'anode, et une seconde résistance de mesure branchée en série entre la cathode et la masse, la mesure de la tension aux bornes des résistances de mesure permettant la déduction des valeurs du courant respectivement à l'anode et à la cathode.
- le dispositif de mesure est en outre adapté pour mesurer simultanément la valeur du courant à l'anode et à la cathode, et pour comparer la valeur du courant dans l'anode et dans la cathode pour détecter l'existence d'un courant de fuite ou d'un courant additionnel dans le corps.

L'invention propose en outre un procédé d'analyse électrophysiologique destiné à être mis en oeuvre dans un système selon l'invention, le procédé comprenant au moins :

    une étape de mesure au cours de laquelle la source de tension continue délivre une série de créneaux de tension continue à une électrode active, et au cours de laquelle le circuit relève des données représentatives du courant dans les électrodes actives et des potentiels sur au moins certaines électrodes connectées en haute impédance, et

    une étape au cours de laquelle on détermine la conductance de la peau au niveau de l'anode, à partir du courant traversant l'anode et de la différence de potentiel entre l'anode et le potentiel atteint par le corps,
    et dans lequel la différence de potentiel entre l'anode et le potentiel atteint par le corps est pilotée par la tension délivrée par la source de tension continue.

Avantageusement, mais facultativement, le procédé selon l'invention comprend en outre la comparaison des valeurs des courants traversant l'anode et la cathode.

BREVE DESCRIPTION DES FIGURES

D'autres caractéristiques, buts et avantages de la présente invention apparaîtront à la lecture de la description détaillée qui va suivre, au regard des figures annexées, données à titre d'exemples non limitatifs et sur lesquelles :

- La figure 1, déjà décrite, représente un exemple de réponse courant-conductance-tension de la peau humaine.
- La figure 2 représente schématiquement un système d'analyse électrophysiologique.
- Les figures 3a et 3b représentent le schéma électrique minimal de deux modes de réalisations d'un système d'analyse électrophysiologique conforme à l'invention.
- Les figures 4a et 4b représentent des exemples de soustracteurs mis en oeuvre dans le système selon l'invention.
- La figure 5 représente les principales étapes d'un procédé d'analyse mis en oeuvre par un système selon l'invention.

DESCRIPTION DETAILLEE D'UN MODE DE REALISATION DE L'INVENTION

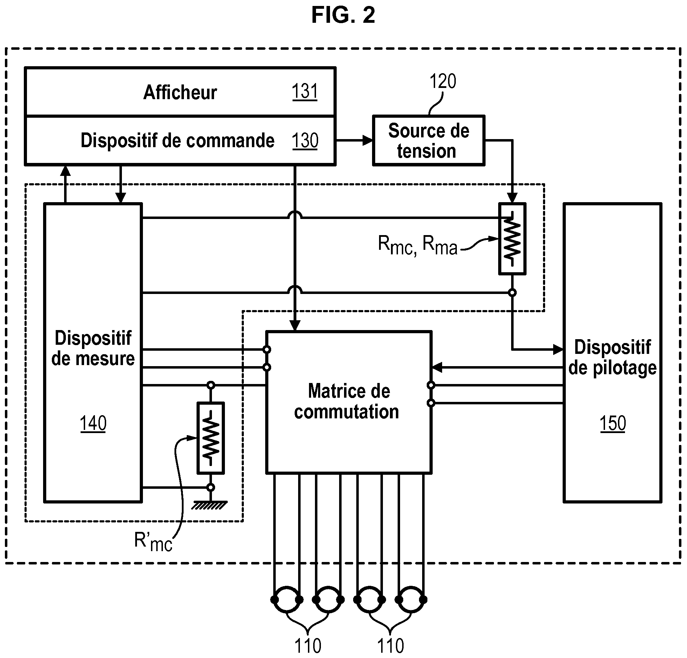

En référence à la figure 2, on a représenté schématiquement un système d'analyse électrophysiologique 100.

Ce système comprend une pluralité d'électrodes 110, de préférence quatre électrodes, parmi lesquelles deux électrodes s'étendent sur une surface suffisamment importante pour qu'un individu puisse y apposer ses mains, et les deux autres électrodes s'étendant sur une surface suffisamment grande pour qu'un individu puisse y poser ses pieds.

Par exemple, ces électrodes peuvent présenter une surface supérieure à 100 cm$^2$.

Le système 100 comprend une source de tension continue 120, adaptée pour générer des créneaux de tension continue. La tension délivrée par la source est de préférence comprise entre 0 et 10 V, avantageusement entre 0 et 4 V. Les créneaux de tension peuvent présenter une durée supérieure ou égale à 0.2 seconde, et varier en tension d'un créneau à l'autre de façon croissante, décroissante ou autre (par exemple croissante puis décroissante).

Le système 100 comprend également un dispositif de commande 130 de la source de tension continue 120 et des électrodes 110. Ce dispositif permet de connecter sélectivement une paire d'électrodes, formant une anode et une cathode, à la source de tension continue pour que celle-ci leur applique des créneaux de tensions. Ces électrodes sont alors dites « actives ».

Les autres électrodes sont alors connectées en haute impédance directement ou indirectement via un amplificateur opérationnel (AO) comme décrit ci-après, et permettent de mesurer le potentiel atteint par le corps. Elles sont appelées électrodes « passives ».

On a représenté dans la figure 2 une matrice de commutation pour illustrer la fonctionnalité de connexion sélective des électrodes à la source de tension continue 120 par le dispositif de commande 130.

Le dispositif de commande 130 peut mettre en oeuvre des cycles de mesure en faisant varier les paires d'électrodes actives et passives. Typiquement, avec un système à quatre électrodes tel que décrit ci-dessus, on effectue les mesures avec les paires d'électrodes suivantes (désignation abrégée entre parenthèses) :

| Anode | Cathode |
|---|---|
| Main gauche (MG) | Main droite (MD) |
| Main droite (MD) | Main gauche (MG) |
| Pied gauche (PG) | Pied droit (PD) |
| Pied droit (PD) | Pied gauche (PG). |

Le système 100 comprend également un dispositif de mesure 140, qui est agencé pour relever les potentiels des électrodes passives, et pour mesurer le courant entre les électrodes actives. A cet égard, le dispositif de mesure 140 comprend avantageusement une résistance de mesure $R_{mc}$ connectée en série entre la cathode et une tension de référence, par exemple la masse. Le courant traversant la cathode est déterminé en mesurant la tension aux bornes de la résistance, et en divisant cette tension par la valeur de la résistance.

Les données mesurées sont affichées sur un dispositif d'affichage 131.

En outre, le système comprend un dispositif de pilotage 150 de la différence de potentiel entre le potentiel $V_a$ de l'anode et le potentiel $V_x$ atteint par le corps, en fonction de la tension délivrée par la source de tension continue. Ce dispositif de pilotage comprend un circuit amplificateur entre la source de tension continue et l'une des électrodes actives, avec une boucle de rétroaction impliquant au moins une des électrodes passives pour contrôler le gain de l'amplificateur et permettre le pilotage de la différence de potentiel entre le potentiel de l'anode et le potentiel du corps Va - Vx.

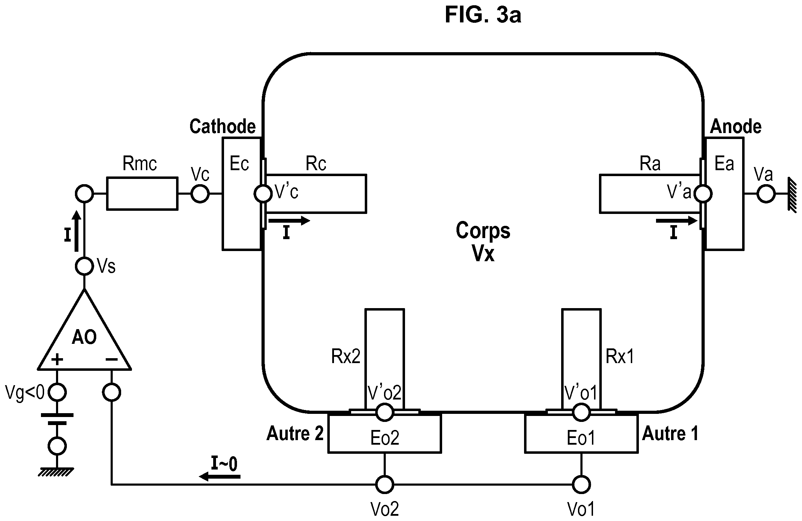

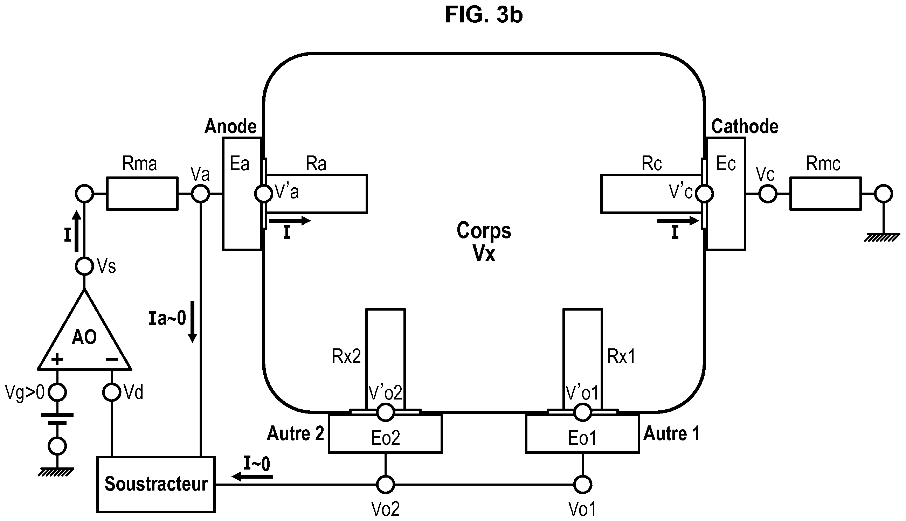

Ce dispositif de pilotage peut faire l'objet de plusieurs réalisations, explicitées ci-après en référence aux figures 3a et 3b.

Selon un premier mode de réalisation, en référence à la figure 3a, l'anode est connectée à la masse, et le circuit amplificateur comprend un amplificateur opérationnel AO, dont l'entrée non-inverseuse est connectée à la source de tension continue $V_g$ négative, dont l'entrée inverseuse reçoit le potentiel Vx atteint par le corps, et dont la sortie $V_s$ est connectée à la cathode à travers la résistance de mesure $R_{mc}$.

Le dispositif de pilotage peut en outre comprendre un dispositif (non représenté sur la figure) pour sélectionner le potentiel atteint par le corps, en fonction des potentiels des électrodes connectées en haute impédance $Vo_1$ et $Vo_2$.

De préférence, le potentiel sélectionné est le minimum de $Vo_1$ et $Vo_2$. Le fait de sélectionner la plus faible valeur de potentiel sur les électrodes connectées en haute impédance permet de s'affranchir des éventuelles surtensions des électrodes en haute impédance.

Comme visible sur la figure 3a, la résistance de mesure $R_{mc}$ du dispositif de mesure 140 est positionnée entre la sortie $V_s$ de l'amplificateur opérationnel et la cathode $V_c$.

Grâce à ce montage, on obtient l'équation du gain :

$$V_s = A(V_g - V_x),$$

où A est le gain de l'amplificateur opérationnel, qui est très supérieur à 1 (par exemple de l'ordre de plusieurs centaines de milliers ou du million).

Les relations sur le courant I traversant le circuit sont :

$$I = \frac{-V_s}{R_{mc} + R_c + R_a} = \frac{-V_x}{R_a}$$

En posant β le taux de rétroaction de la boucle de rétroaction :

$$\beta = \frac{R_a}{R_a + R_c + R_{mc}}$$

où $R_a$ et $R_c$ sont respectivement les résistances des glandes sudorales de la peau coté anode et coté cathode (que l'on cherche à déterminer), on a donc :

$$V_x = \beta . V_s.$$

Et ensuite en éliminant Vs par l'équation du gain, l'équation du pilotage:

$$V_x = \frac{A.\beta}{1+A.\beta}.V_g \approx V_g \text{ car A>>1.}$$

Ainsi, le potentiel Vx atteint par le corps est imposé par le potentiel $V_g$ délivré par la source de tension continue. De plus, comme l'anode est connectée à la masse, son potentiel est nul, et la différence de potentiel $V_a-V_x$ entre l'anode et le potentiel atteint par le corps est égale à $-V_g$, elle est donc pilotée par la source de tension.

On peut de cette manière étudier le comportement de la courbe de réponse tension-courant des glandes sudorales de tous les sujets pour une ddp $V_a-V_x$ constante.

En référence à la figure 3b, on a représenté un mode de réalisation alternatif d'un système muni d'un dispositif de pilotage de la différence de potentiel $V_a-V_x$ entre l'anode et le potentiel atteint par le corps.

Dans ce mode de réalisation, la boucle de rétroaction du dispositif de pilotage comprend un soustracteur recevant sur ses entrées le potentiel de l'anode $V_a$ et le potentiel atteint par le corps $V_x$.

Le circuit amplificateur du dispositif de pilotage comprend un amplificateur opérationnel AO dont l'entrée non-inverseuse est connectée à la source de tension continue délivrant une tension positive, dont l'entrée inverseuse est connectée à la sortie $V_d$ du soustracteur, et dont la sortie est connectée à l'anode à travers la résistance de mesure Rma.

Le soustracteur est en outre adapté pour présenter une impédance d'entrée suffisamment forte pour que les courants d'entrée dans ledit soustracteur ne perturbent pas les mesures, c'est-à-dire pour que le courant I entre l'anode et la cathode ne soit pas biaisé par la présence du soustracteur.

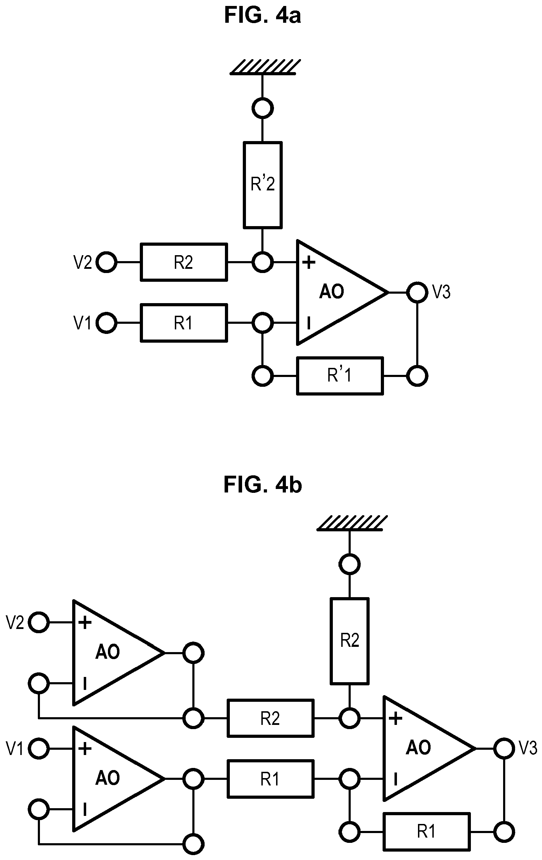

Pour ce faire, le soustracteur peut être réalisé selon différentes manières représentées en figure 4a et 4b.

En référence à la figure 4a, on a représenté un schéma électrique de soustracteur classique réalisé par un amplificateur opérationnel. Dans ce mode de réalisation, la sortie V3 de l'amplificateur opérationnel est reliée à l'entrée inverseuse par une résistance R'1. Une résistance R1 est montée en série entre la première entrée V1 du soustracteur et l'entrée inverseuse.

Enfin, une résistance R2 est montée en série entre la deuxième entrée V2 du soustracteur et l'entrée non-inverseuse de l'amplificateur opérationnel, cette entrée étant également reliée à la masse par une autre résistance R'2.

On obtient donc l'expression de la sortie V3 en fonction des entrées V1 et V2 :

$$V3 = \frac{R1+R'1}{R2+R'2}.\frac{R'2}{R1}.V2 - \frac{R'1}{R1}.V1$$

On obtient, si R1=R'1 et si R2=R'2, le résultat V3=V2-V1.

De plus, si R1 et R2 sont très supérieures à 1, alors les courants prélevés du circuit $i_o$ et $i_a$ sont quasiment nuls.

Un autre mode de réalisation du soustracteur est représenté en figure 4b. Il s'agit du même montage que précédemment, mais dans lequel un amplificateur opérationnel supplémentaire monté en suiveur est placé entre chaque entrée V1, V2 et la résistance correspondante R1, R2.

Dans un suiveur, la sortie Vs de l'amplificateur opérationnel est reliée à la borne inverseuse, et l'entrée est branchée sur la borne non-inverseuse. La sortie est liée à l'entrée V1 par les relations suivantes :

$$V_s = A(V1 - V_s) \text{ et}$$

$$V_s = \frac{A.V1}{1+A} \approx V1 \text{ car A>>1.}$$

L'avantage procuré par ce montage est qu'il permet au niveau du dispositif de pilotage de ne soutirer que des courants négligeables $i_o$ et $i_a$ du dispositif de mesure (inférieurs à $0,1\mu A$) et ceci quelles que soient les valeurs des résistances.

De retour à la figure 3b, les équations de fonctionnement du montage sont les suivantes :

$$V_s = A.\left[V_g - (V_a - V_x)\right]$$

où A est le gain de l'amplificateur opérationnel, et

$$I = \frac{V_s}{R_{ma}+R_a+R_c+R_{mc}} = \frac{V_a-V_x}{R_a}.$$

Dans cette équation, $R_{mc}$ est une première résistance de mesure branchée en série entre la cathode et la masse, et $R_{ma}$ est une seconde résistance de mesure branchée en série entre la sortie Vs de l'amplificateur opérationnel et l'anode. Cette deuxième résistance de mesure est optionnelle. Elle permet de mesurer le courant au niveau de l'anode et au niveau de la cathode pour vérifier qu'il n'y a pas eu de fuite dans le système, et que les courants prélevés par le soustracteur sont nuls.

En posant $\alpha$ le taux de rétroaction de ce système, on a :

$$\alpha = \frac{R_a}{R_{ma} + R_a + R_c + R_{mc}}$$

et

$$V_s = (V_a - V_x)/\alpha.$$

Il en résulte :

$$V_a - V_x = \frac{A.\alpha}{1 + A.\alpha}.V_g \approx V_g$$

car A>>1.

Avec ce circuit, la tension délivrée par la source de tension continue impose directement la différence de potentiel entre le potentiel de l'anode et le potentiel atteint par le corps.

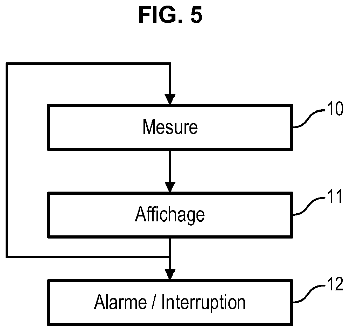

On a présenté en figure 5 les principales étapes du procédé mis en oeuvre avec le système d'analyse présenté ci-avant.

Ce procédé comprend au moins une étape de mesure 10, au cours de laquelle la source de tension continue délivre des créneaux à une électrode active, deux autres électrodes étant connectées en haute impédance. Pendant cette étape, le dispositif de pilotage 150 permet de piloter la valeur de la différence de potentiel Va - VX entre le potentiel de l'anode et le potentiel atteint par le corps en fonction de la source de tension.

Si le circuit comprend deux résistances de mesure, le procédé peut en outre comprendre une étape 11 au cours de laquelle on compare les valeurs du courant à l'anode et à la cathode, en vue de détecter la présence de courants de fuite.

Le résultat de la comparaison peut être affiché sur l'afficheur 131. Si un courant de fuite est détecté, la mesure peut être interrompue ou une alerte déclenchée au cours d'une étape 12.

Grâce au système d'analyse selon l'invention, on peut ensuite exploiter les mesures à différence de potentiel $V_a$-$V_x$ constante pour procéder à l'analyse des décollements des courbes tension-courant de la peau de différents sujets.

**Revendications**

1. Système d'analyse électrophysiologique (100) comprenant :

   une série d'électrodes (110) destinées à être placées en différentes régions
   du corps humain,
   une source de tension continue (120),
   un dispositif de commande (130), adapté pour sélectivement appliquer à une paire d'électrodes dites actives des créneaux de tension continue générés par la source de tension (120), lesdites électrodes actives constituant une anode et une cathode, et pour connecter au moins une autre électrode, dite passive, en haute impédance, ladite électrode servant à mesurer le potentiel atteint par le corps, et
   un dispositif de mesure (140) agencé pour relever des données représentatives du courant à la cathode et des potentiels sur au moins certaines électrodes connectées en haute impédance en réponse à l'application des créneaux, lesdites données permettant de déterminer une valeur de la conductance électrochimique de la peau,
   le système étant **caractérisé en ce qu'**il comprend en outre un dispositif de pilotage (150) de la différence entre le potentiel de l'anode et le potentiel atteint par le corps, en fonction de la tension délivrée par la source de tension continue, et **en ce que** le dispositif de pilotage (150) comprend un circuit amplificateur entre la source de tension continue (120) et l'une des électrodes actives, avec une boucle de rétroaction contrôlant son gain.

2. Système d'analyse électrophysiologique selon la revendication 1, dans lequel l'anode est connectée à la masse, et le circuit amplificateur comprend un amplificateur opérationnel dont :

   l'entrée inverseuse reçoit le potentiel atteint par le corps,
   l'entrée non-inverseuse est connectée à une source de tension continue négative, et
   la sortie est connectée à la cathode.

3. Système d'analyse électrophysiologique selon la revendication 1, dans lequel la boucle de rétroaction du circuit amplificateur comprend un soustracteur recevant sur ses entrées le potentiel de l'anode et le potentiel atteint par le corps, et le circuit amplificateur à boucle de rétroaction comprend un amplificateur opérationnel dont :

   l'entrée inverseuse est connectée à la sortie du soustracteur,
   l'entrée non-inverseuse est connectée à une source de tension continue positive, et
   la sortie est connectée à l'anode.

4. Système d'analyse électrophysiologique selon l'une des revendications précédentes, lequel comprend en outre un dispositif pour déterminer le potentiel atteint par le corps en fonction des potentiels des électrodes connectées en haute impédance.

5. Système d'analyse électrophysiologique selon l'une des revendications 2 et 3, dans lequel le soustracteur présente une impédance d'entrée suffisamment forte, pour que les courants d'entrée dans ledit soustracteur ne perturbent pas les mesures.

**6.** Système d'analyse électrophysiologique selon l'une des revendications 3 à 5, comprenant une première résistance de mesure branchée en série entre la source de tension et l'anode, et une seconde résistance de mesure branchée en série entre la cathode et la masse, la mesure de la tension aux bornes des résistances de mesure permettant la déduction des valeurs du courant respectivement à l'anode et à la cathode.

**7.** Système d'analyse électrophysiologique selon l'une des revendications 1 à 6, dans lequel le dispositif de mesure (140) est en outre adapté pour mesurer simultanément la valeur du courant à l'anode et à la cathode, et pour comparer la valeur du courant dans l'anode et dans la cathode pour détecter l'existence d'un courant de fuite ou d'un courant additionnel dans le corps.

**8.** Procédé d'analyse électrophysiologique destiné à être mis en oeuvre dans un système selon l'une des revendications précédentes, le procédé comprenant au moins :

une étape de mesure au cours de laquelle la source de tension continue (120) délivre une série de créneaux de tension continue à une électrode active, et au cours de laquelle le circuit relève des données représentatives du courant dans les électrodes actives et des potentiels sur au moins certaines électrodes connectées en haute impédance, et

une étape au cours de laquelle on détermine la conductance de la peau au niveau de l'anode, à partir du courant traversant l'anode et de la différence de potentiel entre l'anode et le potentiel atteint par le corps,

et dans lequel la différence de potentiel entre l'anode et le potentiel atteint par le corps est pilotée par la tension délivrée par la source de tension continue.

**9.** Procédé selon la revendication 8, destiné à être mis en oeuvre dans un système selon l'une des revendications 6 ou 7, comprenant en outre la comparaison des valeurs des courants traversant l'anode et la cathode.

**Patentansprüche**

**1.** System zur elektrophysiologischen Analyse (100), umfassend:

eine Reihe von Elektroden (110), die ausgelegt sind, um in verschiedenen Bereichen des menschlichen Körpers platziert zu sein, eine Gleichspannungsquelle (120),

eine Steuervorrichtung (130), die angepasst ist, um selektiv auf ein Paar Elektroden, bezeichnet als aktive Elektroden, Gleichspannungswellen anzulegen, erzeugt durch die Spannungsquelle (120), wobei die aktiven Elektroden eine Anode und eine Kathode darstellen, und um mindestens eine weitere Elektrode, bezeichnet als passive Elektrode, mit hoher Impedanz zu verbinden, wobei die Elektrode dazu dient, das vom Körper erreichte Potenzial zu messen, und eine Messvorrichtung, (140), die angeordnet ist, um repräsentative Daten des Stroms an die Kathode und Potenziale auf mindestens bestimmten Elektroden zu entnehmen, verbunden mit hoher Impedanz in Reaktion auf das Anlegen der Wellen, wobei die Daten ermöglichen, einen Wert der elektrochemischen Konduktanz der Haut zu bestimmen,

wobei das System **dadurch gekennzeichnet ist, dass** es außerdem eine Vorrichtung zur Steuerung (150) der Differenz zwischen dem Potenzial der Anode und dem Potenzial, das vom Körper erreicht wird, in Funktion der Spannung umfasst, die von der Gleichstromquelle geliefert wird, und dadurch, dass die Vorrichtung zur Steuerung (150) eine Verstärkerschaltung zwischen der Gleichstromquelle (120) und einer der aktiven Elektroden umfasst, wobei eine Rückkoppelungsschleife ihre Verstärkung kontrolliert.

**2.** System zur elektrophysiologischen Analyse nach Anspruch 1, wobei die Anode mit der Masse verbunden ist und die Verstärkerschaltung einen Betriebsverstärker umfasst, dessen:

Dreheingang das Potenzial erhält, das vom Körper erreicht wird,
Nicht-Dreheingang mit einer negativen Gleichspannungsquelle verbunden ist, und
Ausgang mit der Kathode verbunden ist.

**3.** System zur elektrophysiologischen Analyse nach Anspruch 1, wobei die Rückkoppelungsschleife der Verstärkerschaltung eine Substrahiervorrichtung umfasst, die auf ihren Eingängen das Potenzial der Anode und das Potenzial, das vom Körper erreicht wird, empfängt, die Verstärkerschaltung mit Rückkoppelungsschleife einen Betriebsverstärker umfasst, dessen:

Dreheingang mit dem Ausgang der Substrahiervorrichtung verbunden ist,
Nicht-Dreheingang mit einer positiven Gleichspannungsquelle verbunden ist, und
Ausgang mit der Anode verbunden ist.

**4.** System zur elektrophysiologischen Analyse nach einem der vorhergehenden Ansprüche, das außerdem eine Vorrichtung umfasst, um das Potenzial, das vom Körper erreicht wird, in Funktion der Potenziale der Elektroden zu bestimmen, die mit hoher Impedanz verbunden sind.

5. System zur elektrophysiologischen Analyse nach einem der Ansprüche 2 und 3, wobei die Substrahiervorrichtung eine Eingangsimpedanz aufweist, die ausreichend stark ist, damit die Eingangsströme in der Substrahiervorrichtung die Messungen nicht stören.

6. System zur elektrophysiologischen Analyse nach einem der Ansprüche 3 bis 5, umfassend eine erste Messresistenz, die in Reihe zwischen der Spannungsquelle und der Anode unter Strom gesetzt ist, und eine zweite Messresistenz, die in Reihe zwischen der Kathode und der Masse unter Strom gesetzt ist, wobei die Messung der Spannung an den Klemmen der Messresistenzen die Ableitung der Werte des Stroms an der Anode bzw. an der Kathode ermöglicht.

7. System zur elektrophysiologischen Analyse nach einem der Ansprüche 1 bis 6, wobei die Messvorrichtung (140) außerdem angepasst ist, um gleichzeitig den Wert des Stroms an der Anode und an der Kathode zu messen, und um den Wert des Stroms in der Anode und in der Kathode zu vergleichen, um die Existenz eines Leckstroms oder eines Zusatzstroms im Körper nachzuweisen.

8. Verfahren zur elektrophysiologischen Analyse, die ausgelegt ist, um in einem System nach einem der vorhergehenden Ansprüche durchgeführt zu werden, wobei das Verfahren mindestens Folgendes umfasst:

einen Schritt des Messens, im Laufe dessen die Gleichspannungsquelle (120) eine Reihe von Gleichspannungswellen an eine aktive Elektrode liefert, und im Laufe dessen die Schaltung repräsentative Daten des Stroms in den aktiven Elektroden und Potenziale auf mindestens bestimmten Elektroden, verbunden mit hoher Impedanz, entnimmt, und
einen Schritt, im Laufe dessen die Konduktanz der Haut auf dem Niveau der Anode bestimmt wird, ausgehend vom Strom, der die Anode quert, und der Potenzialdifferenz zwischen der Anode und dem Potenzial, das vom Körper erreicht wird,
und wobei die Potenzialdifferenz zwischen der Anode und dem Potenzial, das vom Körper erreicht wird, durch die Spannung gesteuert wird, die von der Gleichspannungsquelle geliefert wird.

9. Verfahren nach Anspruch 8, das ausgelegt ist, um in einem System nach einem der Ansprüche 6 oder 7 durchgeführt zu werden, umfassend außerdem den Vergleich der Werte der Ströme, die die Anode und die Kathode queren.

**Claims**

1. Electrophysiological analysis system (100) comprising:

a series of electrodes (110) intended to be placed
in different regions of the human body,
a direct current voltage source (120),
a control device (130), suitable for selectively applying direct current pulses, generated by the voltage source (120), to a pair of so-called active electrodes, said active electrodes forming an anode and a cathode, and for connecting at least one other so-called passive electrode with high impedance, said electrode serving to measure the potential reached by the body, and
a measurement device (140) arranged to obtain data representative of the current at the cathode and the potentials on at least some of the electrodes connected with high impedance, in response to the application of the pulses, said data being suitable for determining a value of the electrochemical conductance of the skin,
the system being **characterised in that** it further comprises a device (150) for controlling the difference between the potential of the anode and the potential reached by the body, as a function of the voltage delivered by the direct current voltage source, and **in that** the control device (150) comprises an amplifier circuit between the direct current voltage source (120) and one of the active electrodes, with a feedback loop controlling the gain thereof.

2. Electrophysiological analysis system according to claim 1, wherein the anode is connected to the ground, and the amplifier circuit comprises an operational amplifier wherein:

the inverting input receives the potential reached by the body,
the non-inverting input is connected to a negative direct current voltage source, and
the output is connected to the cathode.

3. Electrophysiological analysis system according to claim 1, wherein the feedback loop of the amplifier circuit comprises a subtractor receiving on the inputs thereof the potential of the anode and the potential reached by the body, and the feedback loop amplifier circuit comprises an operational amplifier wherein:

> the inverting input is connected to the subtractor output,
> the non-inverting input is connected to a positive direct current voltage source, and
> the output is connected to the anode.

4. Electrophysiological analysis system according to one of the preceding claims, further comprising a device for determining the potential reached by the body as a function of the potentials of the electrodes connected with high impedance.

5. Electrophysiological analysis system according to one of claims 2 and 3, wherein the subtractor has a sufficiently high input impedance, so that the input currents in said subtractor do not disturb the measurements.

6. Electrophysiological analysis system according to claims 3 to 5, comprising a first measurement resistor connected in series between the voltage source and the anode, and a second measurement resistor connected in series between the cathode and the ground, the voltage measurement at the terminals of the measurement resistors being suitable for inferring the values of the current at the anode and at the cathode respectively.

7. Electrophysiological analysis system according to one of claims 1 to 6, wherein the measurement device (140) is further suitable for simultaneously measuring the value of the current at the anode and at the cathode, and for comparing the value of the current in the anode and in the cathode to detect the existence of a leakage current or an additional current in the body.

8. Electrophysiological analysis method intended to be implemented in a system according to any of the above claims, the method comprising at least:

> a measurement step during which the direct current voltage source (120) delivers a series of direct current pulses to an active electrode, and during which the circuit collects data representative of the current in the active electrodes and potentials on at least some electrodes connected with high impedance, and
> a step during which the conductance of the skin at the anode is determined, on the basis of the current passing through the anode and the difference in potential between the anode and the potential reached by the body,
> and wherein the difference in potential between the anode and the potential reached by the body is controlled by the voltage delivered by the direct current voltage source.

9. Method according to claim 8, intended to be implemented in a system according to any of claims 7 or 8, further comprising the comparison of the values of the currents passing through the anode and the cathode.

# FIG. 1

EP 2 890 294 B1

# FIG. 2

Afficheur 131

Dispositif de commande 130

Source de tension 120

$R_{mc}, R_{ma}$

Dispositif de mesure 140

Matrice de commutation

Dispositif de pilotage 150

$R'_{mc}$

110    110

EP 2 890 294 B1

# FIG. 3a

EP 2 890 294 B1

FIG. 3b

## FIG. 4a

## FIG. 4b

# FIG. 5

## RÉFÉRENCES CITÉES DANS LA DESCRIPTION

**Documents brevets cités dans la description**

- FR 2912893

- FR 1160601

**Littérature non-brevet citée dans la description**

- **GIN H et al.** Non-invasive and quantitative assessment of sudomotor function for peripheral diabetic neuropathy evaluation. *Diabetes Metab,* 2011